# EUROPEAN PATENT SPECIFICATION

(11) **EP 2 180 850 B1**
(45) Date of publication and mention of the grant of the patent: **21.11.2018**
(21) Application number: 08776653.1
(22) Date of filing: 24.07.2008
(51) Int. Cl.: A61L 27/56, A61L 27/14, A61L 24/00, A61L 24/04, A61L 27/50

(54) **EXPANDABLE BONE FILLER MATERIALS AND METHODS OF USING SAME**
EXPANDIERBARE KNOCHENFÜLLMATERIALIEN UND ANWENDUNGSVERFAHREN DAFÜR
MATÉRIAUX EXTENSIBLES DE REMPLISSAGE OSSEUX ET LES PROCÉDÉS LES UTILISANT

(30) Priority: 25.07.2007 US 951717 P
(43) Date of publication of application: 05.05.2010
(73) Proprietor: Depuy Spine, Inc., Raynham, MA 02767 (US)
(72) Inventor: BEYAR, Etai, 46724 Herzlia Pituach (IL); BEYAR, Mordechay, 46724 Herzlia Pituach (IL); GLOBERMAN, Oren, 46724 Herzlia Pituach (IL); SHAVIT, Ronen, 69413 Tel-aviv (IL); WACHSLER-AVRAHAMI, Hila, 69400 Tel-aviv (IL)
(74) Representative: Curran, Clair
(86) International application number: PCT/IL2008/001026
(87) International publication number: WO 2009/013752

(56) References cited:
- WO-A1-2005/034781
- WO-A2-03/065996
- WO-A2-03/065996
- WO-A2-2004/073563
- US-A1- 2006 008 504
- US-A1- 2006 241 632
- US-A1- 2006 241 632
- US-B1- 6 383 190
- US-B2- 6 726 691

## Description

### FIELD AND BACKGROUND OF THE INVENTION

The present invention relates to bone cement and, more particularly, but not exclusively, to expandable bone cements and/or fillers.

It is common to employ cement to repair bones in a variety of clinical scenarios. For example, compression fractures of the vertebrae, which are a common occurrence in older persons, cause pain and/or a shortening (or other distortion) of stature. In a procedure known as vertebroplasty cement is injected into a fractured vertebra. Vertebroplasty stabilizes the fracture and reduces pain, although it slightly restore the vertebral height and in rare cases to its original height. In vertebroplasty the cement is typically injected in a liquid phase so that resistance to injection is not too high. US-A-2006/2416 32 discloses an expandable bone cement/bone filler material comprising PMMA and the cement changes from a flowable state to a solid state. Liquid cement may unintentionally be injected outside of the vertebra and/or may leak out through cracks in the vertebra or into blood vessels. Such a leakage can be dangerous as it can harm adjacent nerves and/or other tissue.

In another procedure, known as Kyphoplasty, the fracture is reduced by expanding a device, such as a balloon, inside the vertebra and then injecting the cement. Kyphoplasty reduces the risk of cement leakage by permitting a lower pressure to be used for injection of the cement, as the cement is injected into a pre-dilated void.

US Application Publication No. 2007/0032567 teaches of a new type of bone filler material (commercially available as "Confidence Bone Cement™" by Disc-O-Tech Medical Technologies, Ltd.; Israel) having no liquid phase and preserving a relatively stable high viscosity for several minutes immediately after mixing. These main characteristics provide a substantially safer filler material for vertebroplasty procedures with less risk of leakage, and further provide some height restoration in specific cases of Vertebral Compression Fractures (VCF).

US Application Publication No. 2006/0122625 to Truckai et al., presents a new method of injecting filler material for treating VCF in which an external energy (e.g., RP) is used to change a material flow property (e.g., viscosity) during injection and/or in between two sequential injections. In an embodiment, a first volume of lower viscosity filler is injected to the vertebra, then RF energy is emitted to enlarge the first volume filler viscosity, and finally a second volume of same filler is injected into to first volume, which now may serve as an expandable outer cover, which may improve both leakage durability and height restoration.

### SUMMARY OF THE INVENTION

The invention provides an expandable bone filler material, as defined in claim 1.

The scope of the invention is defined by the claims. Any references in the description to methods of treatment refer to the products of the present invention for use in a method for treatment. Optionally, the expandable bone filler material is adapted to engage and/or interface with bones located at the injection site as the material expands into orifices and/or pores of the bone during and/or after injection. Optionally, the expandable bone filler material enables low pressure injection during treatment reducing the chance of undesirable leakage of the filler material at the injection site. Compression fractures may be treated with the expandable bone filler material wherein expansion of the material decompresses the fracture.

In an embodiment of the invention, air pockets and/or bubbles within the filler material expand when heated, thereby causing the filler material as a whole to expand. In some embodiments of the invention, air pockets are temporarily encased in a degradable material, such as polyglycolic acid (PGA) which, when the degradable material breaks down, the air is released resulting in an expansion of the bone filler material matrix. Optionally, the degradable material is biocompatible and/or biodegradable. In some embodiments of the invention, the degradable material has a relatively low Tg and when the bone filler material is heated, but still in the liquid phase, the air is slowly released from the degradable material expanding the overall bone filler material matrix.

In an embodiment of the invention, air pockets within the filler material are created during the injection process, wherein an injection device is used which imparts air pockets to the expandable filler material as it is being injected. Optionally, a cannula with at least one side exit port is used as the injection device. Optionally, a fenestrated cannula is used as the injection device such that injected filler material is injected as a plurality of separate extrusions which aggregate after injection, trapping air pockets therebetween.

In an embodiment of the invention, applied energy is at least one of heat, radiofrequency (RF), light (coherent and/or broadband), ultrasound, microwave, electrical and/or magnetic based. Optionally, applied light energy is laser and/or infrared based. In some embodiments of the invention, at least a portion of the applied heat energy is from the exothermic reaction of the curing process of the bone filler material. In some embodiments of the invention, a component or additive is added to the bone filler material depending on the type of energy to be applied for expansion activation. For example, wire coils are included in the bone filler material for E-M radiation activation and/or air pockets are included in the bone filler material for an expansion response to heat and/or ultrasound.

Optionally, at least one of the bone filler material components or additives produces and/or discharges a gas that promotes bone filler material expansion. Optionally, gas discharge occurs at a predetermined temperature and/or time- from-mixing. In an embodiment of the invention, a biodegradable material which releases CO2 is used as a gas discharging additive.

Optionally, the expandable bone filler material is adapted to expand at a predetermined time. In some embodiments of the invention, expansion of the bone filler material commences upon mixing the material together. In some embodiments of the invention, the bone filler material does not expand until after a predetermined amount of time. In an exemplary embodiment of the invention, the bone filler material starts expansion during the "working phase", the working phase being when the bone filler material is liquid and/or doughy so it can be manipulated into bone and/or interdigitate within a cancellous bone. In an exemplary embodiment of the invention, the bone filler material starts expansion during the "setting phase", the setting phase being when the bone filler material accelerates its curing to achieve full hardness. Optionally, the bone filler material starts expanding during the working phase and continues expansion into and after the setting phase. In an embodiment of the invention, the bone filler material does not experience expansion until after a predetermined time after the setting phase begins. In some embodiments of the invention, the bone filler material is set between 10 and 45 minutes from the start of mixing.

Optionally, the filler material expands after a predetermined period of time from mixing start.

Optionally, the expansion time is adapted to overlap for a predetermined time the working phase and/or setting phase of the filler material. A predetermined expansion time and/or amount are used to provide guidelines and/or parameters for the timing of the treatment procedure, in an embodiment of the invention.

Optionally, a specific quantity and/or mass of the bone filler material is adapted to expand a predetermined amount and/or over a predetermined period of time.

Optionally, the expandable bone filler is adapted for treating a vertebral compression fracture.

Bone filler materials of the invention can be used in methods of treating vertebral compression fractures by injecting the expandable bone filler material in a position to decompress the compression fracture by expanding. An optional "design" phase for planning performance defining characteristics including at least one of the location of injection and/or duration of expansion and/or time of expansion start/end and/or amount of expansion and/or direction(s) of expansion and/or speed of expansion and/or viscosity (which is optionally variable over time, in an embodiment of the invention) is included in the treatment method. The designing may occur before injection.

Optionally, the bone material filler is mixed at or near the start of the treatment procedure. Optionally, mixing includes providing an additive to the bone filler mix which will provide an expansion force to at least a portion of the mixture. A component of the mix provides the expandable quality to at least a portion of the mixture.

Optionally, the bone filler mixture is injected into a treatment site using an injection device adapted to cause and/or assist the bone filler expansion. A cannula may be used as an injection device. Optionally, the cannula is a fenestrated cannula which creates air pockets within the bone filler material during the injection process.

Expansion of the bone filler material may be directed and/or designed. For example, the bone filler material is injected in situ in a specific direction and/or location. Optionally, the bone filler material is injected in a specific direction and/or location using a cannula as the injection device. Expansion of the bone filler material may be adapted during the design process to account for the patient being treated by a pressure relief mechanism, such as traction, reducing the amount of resistance imparted to the expansion mechanism of the bone filler material.

Optionally, expandable additive and/or component is located in some portions of the bone filler material but not in other portions, such that when expansion of the additive occurs, at least direction and/or amount of expansion is controlled. Optionally, expansion is pre-designed by embedding in the bone filler material an expandable body, such as a sponge, which is adapted to expand in a specific direction or directions. Optionally, selective application of energy to the bone filler mixture causes energized portions of the bone filler mixture to expand more than non-energized portions.

A compressed vertebra may be treated using the methodology described herein.

Bone filler materials of the invention can be used in a method of performing vertebroplasty while using a low injection pressure for enhanced safety. Optionally, the expandable quality of the bone filler material obviates the need for injection at high pressure, which is normally used to disperse the bone filler material throughout the treatment site and/or into extensive contact with the bone structure surrounding the treatment site. Optionally, "low" pressure means less than 1.5 MPa (15 bar). Optionally, low pressure is between 0.8 Mpa (8 bar) and 1.5 MPa (15 bar). Optionally, low pressure is less than 1 MPa (10 bar). Expanding the bone filler material at least partially increases the height of the bone structure surrounding the treatment site.

Bone filler materials of the invention can be used in a method of kyphoplasty at a low injection pressure and/or without using a kyphoplasty balloon. Optionally, "low" pressure means 1.5 MPa (15 bar) or less. Optionally, low pressure is between 0.8 Mpa (8 bar) and 1.5 MPa (15 bar). Optionally, low pressure is less than 1 MPa (10 bar). The bone filler material expands to stabilize and/or increase the height of a bone structure surrounding the treatment site and/or to provide extensive surface area contact between the bone filler material and the bone structure.

Expandable bone filler material may be injected into a treatment site without effectuating height restoration to the bone structure surrounding the treatment site, for example providing stabilization and/or pain relief without height restoration.

In an embodiment of the invention, the volume increases by at least 5%. Optionally, the volume increases by at least 10%. Optionally, the volume increases by at least 20%. Optionally, the volume increases by at least 50%. Optionally, the volume increases by at least 100%.

In an embodiment of the invention, at least a portion of the at least one additive contributes to expansion as a result of having energy applied to the at least one additive. In some embodiments of the invention, energy is at least one of heat, radiofrequency, light, ultrasound, microwave, electrical or magnetic. Optionally, light is at least one of coherent or broadband.

In an embodiment of the invention, the at least one additive is air. Optionally, the air is placed in the bone filler material by gas discharging from the bone filler material. Optionally, the air is placed into the bone filler material by an injection device during injection into the treatment site.

In some embodiments of the invention, at least a portion of the applied energy is derived from a curing process of the bone filler material.

In an embodiment of the invention, the at least one component is used to impart at least one performance defining characteristic to the expansion of the material. Optionally, a characteristic is one of duration of expansion, time from mixing of expansion start, time from mixing of expansion end, amount of expansion, direction of expansion, speed of expansion and viscosity. Optionally, at least one performance defining characteristic is variable over time.

In some embodiments of the invention, the material is biocompatible.

Bone filler materials of the invention can be used in a method of using an expandable bone filler material, comprising: expanding at least a portion of the bone filler material, thereby increasing the overall volume of the bone filler material, at a treatment site. Optionally, the method further comprises injecting the expandable bone filler material with at least one component adapted to expand at least a portion of the bone filler material.

Optionally, the method further comprises designing the material to exhibit at least one performance defining characteristic. Optionally, a performance defining characteristic is at least one of duration of expansion, time from mixing of expansion start, time from mixing of expansion end, amount of expansion, direction of expansion, speed of expansion and viscosity. Optionally, the method further comprises embedding an expandable object in the mixture.

Optionally, expanding is activated by applying energy to at least the portion of the bone filler.

Optionally, the method further comprises withdrawing at least one injection device from the treatment site.

Bone filler materials of the invention can be used in a method of at least partially decompressing a vertebral compression fracture using an expandable bone filler material, comprising: injecting the expandable bone filler material at a treatment site, wherein at least a portion of the bone filler material is adapted to expand, increasing the overall volume of the material and decompressing at least partially the compression fracture.

Optionally, the method further comprises designing the material to exhibit at least one performance defining characteristic. Optionally, a performance defining characteristic is at least one of duration of expansion, time from mixing of expansion start, time from mixing of expansion end, amount of expansion, direction of expansion, speed of expansion and viscosity.

Optionally, the method further comprises embedding an expandable object in the material.

Optionally, the method further comprises activating at least the portion of the material adapted to expand by applying energy to the portion. Optionally, applying energy includes applying at least one of heat, radio frequency, light, ultrasound, microwave, electrical or magnetic energy.

Optionally, the method further comprises withdrawing at least one injection device from the treatment site.

Bone filler materials of the invention can be used in a method of performing vertebroplasty, comprising: injecting an expandable bone filler material at a treatment site using an injection pressure less than 1.5 MPa (15 bar); and, expanding the bone filler material at the treatment site to at least stabilize a bone structure surrounding the treatment site. Optionally, the injection pressure is between 0.8-1.5 MPa (8-15 bar). Optionally, expanding the bone filler material at least partially increases a height of the bone structure surrounding the treatment site.

Bone filler materials of the invention can be used in a method of performing kyphoplasty, comprising: injecting an expandable bone filler material at a treatment site using an injection pressure less than 1.5 MPa (15 bar); and, expanding the bone filler material at the treatment to at least stabilize a bone structure surrounding the treatment site, wherein the treatment site is not prepared for receipt of the bone filler material prior to injecting. Optionally, the injection pressure is between 0.8-1.5 MPa (8-15 bar). Optionally, expanding the bone filler material at least partially increases a height of the bone structure surrounding the treatment site.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced. In the drawings:
Fig. 1 is a flowchart depicting a method for using an expandable bone filler;
Fig. 2 is a flowchart depicting a method for treating a compression fracture;
Fig. 3A is a schematic diagram showing expandable bone filler material injected in proximity to a vertebral compression fracture prior to full expansion of the filler;
Fig. 3B is a schematic diagram showing expandable bone filler material injected in proximity to a vertebral compression fracture after at least partial expansion of the filler;
Fig. 4A is an illustration of an expandable, open-cell foam object compressed in an injection device prior to expansion;
Fig. 4B is an illustration of an expandable, open-cell foam object being deployed out of an injection device in a partially expanded form;
Fig. 4C is an illustration of an expandable, open-cell foam object after expansion at a treatment site;
Fig. 5A is an illustration of a fenestrated cannula for injecting bone filler material at a treatment site;
Fig. 5B is a close-up of the fenestrated cannula extruding bone filler material;
Fig. 6A is a cross-sectional view showing bone filler material injected into a treatment site prior to activation of expansion and,
Fig. 6B is a cross-sectional view showing bone filler material being activated at a treatment site.

### DESCRIPTION OF EXEMPLARY EMBODIMENTS OF THE INVENTION

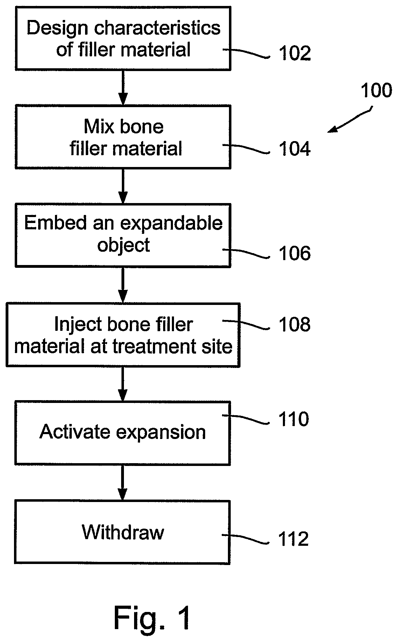

Referring to Fig. 1, a flowchart 100 depicting a method for using an expandable, biocompatible bone filler is shown.

Methods for using an expandable bone filler are adapted to utilize configurable characteristics of expandable bone filler materials. Designing (102) the characteristics of the expandable bone filler material is optionally performed prior to mixing (104) of the material. Performance defining characteristics of the bone filler material, include as examples: duration of expansion; and/or time from mixing of expansion start and/or end; and/or amount of expansion; and/or direction(s) of expansion; and/or speed of expansion; and/or viscosity (which is optionally variable over time.

In addition to the above, the location of injection of the bone filler material is optionally optimized in conjunction with a characteristic of the material, for example, injecting the bone filler material in a specific location such that the bone filler material expands in a predetermined direction from the specific location to have a desired effect on the patient. Optionally, a specific location for injection is determined while designing (102) the characteristics of the bone filler material.

The bone filler material may be adapted (e.g. during designing (102)) to engage interstitial spaces of the bones at the injection site in order to provide a stable and/or supporting bone filler mass once the bone filler material is set. Optionally, the expandable nature of the bone filler material causes the bone filler to expand into the interstitial spaces of the bones at the injection site. An attending medical professional may design the bone filler material mixture on the fly, adjusting at least one of additives and/or components in the mixture, direction and/or amount and/or type of applied energy, and/or injection device used.

Speed of expansion may be designed (102) by adjusting applied energy and/or porosity of the material coating the at least one additive. Optionally, the material coating the additive is the bone filler material. Optionally, the material coating the additive is a part of the additive. Expansion is halted by an inherent property of the additive (e.g. a sponge has a maximum amount it can expand) and/or by limiting the applied energy, for example terminating exposure of the bone filler material to the applied energy. Viscosity may be controlled and/or designed in order to achieve a desired amount of expansion. Optionally, a known viscosity profile of the base components of the bone filler material determines the choice of additive(s) and/or applied energy used.

Expansion of the bone filler material may be adapted during the design (102) process to account for the patient being treated by a pressure relief mechanism, such as traction, reducing the amount of resistance imparted to the expansion mechanism of the bone filler material during expansion at the treatment site.

The bone filler material is acrylic bone cement. The cement may be produced by mixing (104) at least two components, one of which contains a polymethylmethacrylate powder and the other component containing a liquid methylmethacrylate monomer.

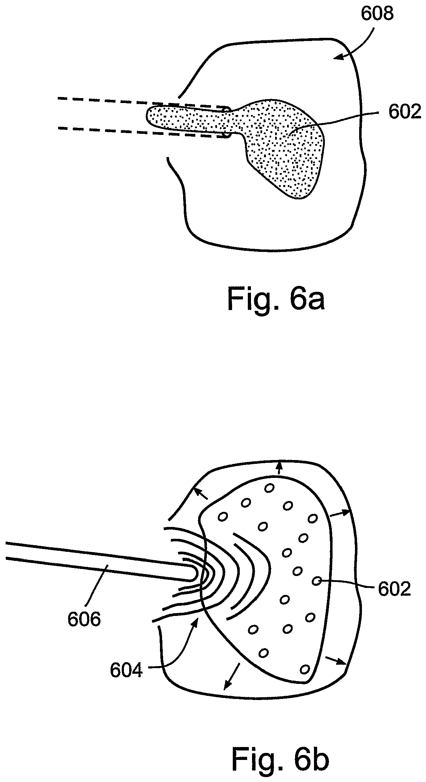

Bone filler material expansion may be accomplished by expansion of at least one component of said filler material and/or a specific region or part of the filler material. Mixing (104) may include adding at least one component or additive 602, shown in Fig. 6A, which is adapted to provide expansive forces to the bone filler material mixture at the treatment site 608. The at least one additive may be radio-opaque. The at least one additive may represent between 5% and 45% of the total volume of the bone filler material mixture. Optionally, the at least one additive represents more than 45% of the total volume of the mixture.

In an embodiment of the invention, the additive expands as a result of energy 604 applied to it, such as described with respect to activation (110) and Fig. 6B, below. In some embodiments of the invention, a component or additive is added to the bone filler material depending on the type of energy to be applied for expansion activation (110). For example, wire coils are included in the bone filler material for E-M radiation activation (110) and/or air pockets are included in the bone filler material for an expansion response to heat and/or ultrasound.

In some embodiments of the invention, the additive discharges a gas which causes expansion of the bone filler material. In an embodiment of the invention, a biodegradable material which releases CO₂ is used as a gas discharging additive.

In some embodiments of the invention, the bone filler material is designed (102) to contain air pockets which, when heated, expand causing the bone filler material to expand in general. In some embodiments of the invention, air pockets are temporarily encased in a degradable material, such as PGA which, when the degradable material breaks down, the air is released resulting in an expansion of the bone filler material matrix. Optionally, the degradable material is biocompatible and/or biodegradable. In some embodiments of the invention, the degradable material has a relatively low Tg (e.g. 36°C-40°C) and when the bone filler material is heated, but still in the liquid phase, the air is slowly released from the degradable material. Air pockets are optionally created in the bone filler material during mixing (104) and/or injection (108), as described in more detail below. Pockets may be added at the treatment site separately from the bone filler material. Optionally, air pockets represent between 10% and 45% of the volume of the bone filler material mixture.

The bone filler material may be designed (102) to expand in a particular spatial fashion. For example, the material may be designed (102) to expand in a specific direction or directions and/or with a particular shape (e.g. spherical expansion).

In an exemplary embodiment of the invention, at least a portion of the bone filler material expands at least 5% from its original volume. Optionally, at least a portion of the bone filler material expands at least 10% from its original volume. Optionally, at least a portion of the bone filler material expands at least 20% from its original volume. Optionally, at least a portion of the bone filler material expands at least 50% from its original volume. Optionally, at least a portion of the bone filler material expands at least 100% from its original volume.

Optionally, the addition of air pockets which represent about 10%-20% by volume of the bone filler material and/or the addition of a sufficient amount of additive, like PGA, to expand the bone filler material by about 10%-15% will diminish the strength of the bone filler material by about 10%-15%.

The bone filler material may start to expand after a preset period of time after mixing (104) starts. Optionally, expansion starts immediately. Optionally, expansion occurs more than 3 minutes after mixing (104) of the filler material components starts. Optionally, expansion occurs more than 5 minutes after mixing (104) of the filler material components starts. Optionally, expansion occurs more than 10 minutes after mixing (104) of the filler material components starts. Optionally, expansion occurs more than 15 minutes after mixing (104) of the filler material components starts. Optionally, expansion occurs a matter of hours or days after the mixing (104) starts, which may be after the bone filler material has been injected into the patient. A predetermined expansion time and/or amount are used to provide guidelines and/or parameters for the timing of the treatment procedure.

Optionally, mixing (104) starts the expansion of the bone filler material, as described elsewhere herein. As the base components of the bone filler material are acrylic, the bone filler material goes through a polymerization process after mixing (104) starts, so the mixed material becomes more viscous over time until it sets to full hardness. Optionally, full hardness is similar to bone hardness. Optionally, bone filler material hardness is softened by at least one additive or component in the mixture. Different compositions may lead to different polymerization behaviors/profiles/characteristics, however all acrylic bone filler materials have at least two main phases after mixing: the "working phase", when the bone filler material is liquid and/or doughy so it can be manipulated into bone and/or interdigitate within a cancellous bone, and the "setting phase", when the bone filler material polymerization accelerates until full hardness. In an exemplary embodiment of the invention, the bone filler material expands after it is introduced into bone and before and/or during its setting phase. In some embodiments of the invention, bone material expansion begins while still in the working phase. Various mixing (104) techniques are optionally used to impart at least expansion promoting property to the bone filler material, for example bone filler material containing entrapped air pockets can be prepared by mixing the material components with air (as in non-vacuum mixers).

It should be understood that additives and/or components are optionally added depending on the desired performance of the bone filler material and that actual formulations of bone filler materials may vary depending on the needs of the patient and/or the opinion of the attending medical professional.

An expandable object or objects may be embedded (106) in the bone filler material. At least one moisture absorbent, expandable open-cell foam object, for example a sponge may be embedded (106) in the bone filler material mixture. Exemplary absorbent materials are described in US Patent No. 6,692,528 to Ward et al. The moisture which is absorbed by the at least one moisture absorbent expandable object comes from the surrounding tissue of the patient's body.

The bone filler material may be introduced into a vertebral body with at least one expandable, optionally initially compressed, object. Optionally, the at least one expandable, optionally initially compressed, object is a sponge. The expandable object(s) is not nominally sufficient by itself to support the structures surrounding the treatment site but when used in combination with the bone filler material and/or with a plurality of objects is sufficient to provide support.

When the object decompresses, it causes expansion in a predetermined and/or designed direction or directions. A plurality of expandable objects, such as sponges and/or compressible balls, may be compressed during injection and then expanded after arriving at the treatment site. Optionally, the initially compressed expandable object is also moisture absorbent. Optionally, the object is soaked and/or saturated with said filler material when embedded (106). Alternatively and/or optionally, the object is introduced separately from the bone filler material into the patient. Optionally, the object is introduced without any or additional bone filler material being introduced into the patient. In some embodiments of the invention, the object is introduced in conjunction with the bone filler material after it has begun to cure, reducing and/or eliminating the amount of bone filler material absorbed by the object. In some embodiments of the invention, bone filler material is used to fix the object to its surroundings within the patient.

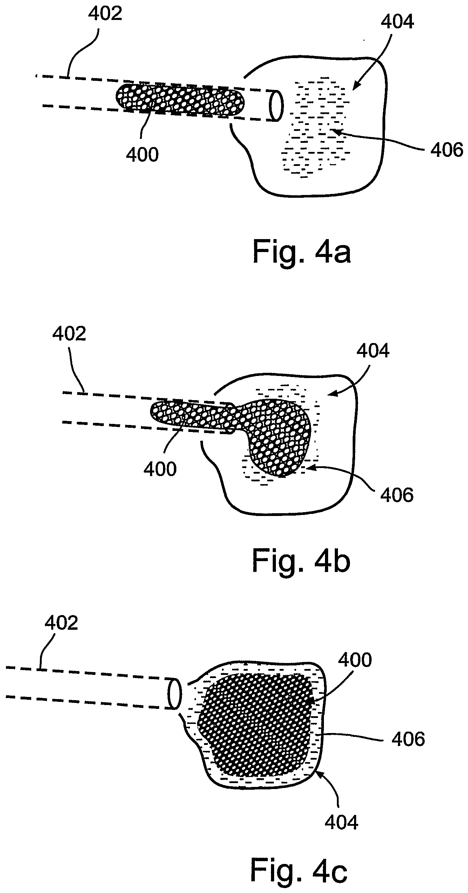

Optionally, the object 400 is introduced to the treatment site 404 via a cannula 402 while in compressed mode (shown in Fig. 4A) and then expands to a larger size, as shown in Figs. 4B-4C. Optionally, a small diameter cannula (for example having 1-5mm diameter, optionally about 3mm diameter) is used. As described above, bone filler material 406 is optionally used in conjunction with the expandable object 400. It should be noted that while Fig. 4C illustrates the object 400 occupying the treatment site 404 almost entirely, optionally, the object 400 expands to only partially occupy the treatment site 404.

In an exemplary embodiment of the invention, at least one of the bone filler material components or additives produces or discharges gas that promotes overall bone filler material expansion. Optionally, gas discharge by the bone filler additive occurs at a predetermined temperature and/or time. Optionally, the time is measured from mixing.

The bone filler material may be injected (108) into a treatment site via an injection device, such as a cannula. The injection device may be used to inject (108) the bone filler material into a specific location and/or in a specific direction at the treatment site. An exemplary injection device having a side exit port for providing directed bone filler material injection (108) is described in US Application Publication no. 2008/0228192.

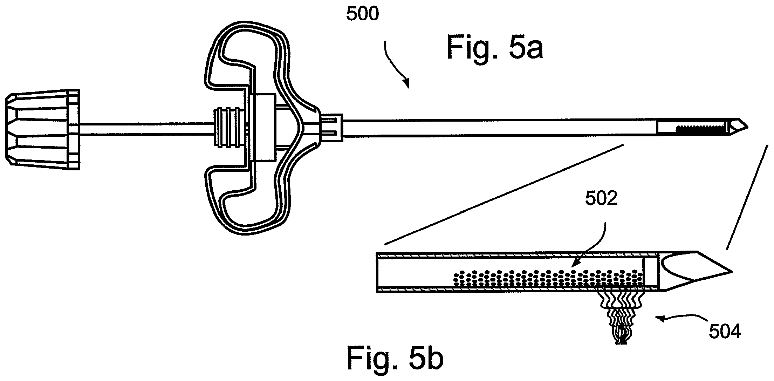

The injection device is optionally used to provide the bone filler material mixture with a desirable property, for example containing air pockets, in an exemplary embodiment of the invention. Optionally, a fenestrated cannula 500, shown in more detail in Figs. 5A and 5B, is used as the injection device for imparting air pockets to the bone filler material mixture. Using a fenestrated cannula, the filler material is injected (108) into the treatment site as separated, thin hair-like extrusions 504 that are aggregated inside the patient at the treatment site, trapping air pockets between the extrusions that have aggregated.

An exemplary fenestrated cannula that is optionally modified for use as an injection device using the herein described methodologies is described in WO-A-2007/122608. In some embodiments of the invention, the fenestrations 502 of the fenestrated cannula is modified to impart air pockets in certain portions of the injected bone cement. Optionally, the fenestrated cannula 500 is adapted to create certain sizes and/or certain parts per volume of air pockets in the bone filler material.

In an embodiment of the invention, the air pockets and/or bubbles expand when activated (110) by heat, thus promoting expansion of the overall bone filler material mass. The injection device may also be used to inject fluids and/or expandable additives and/or components and/or objects with the bone filler material.

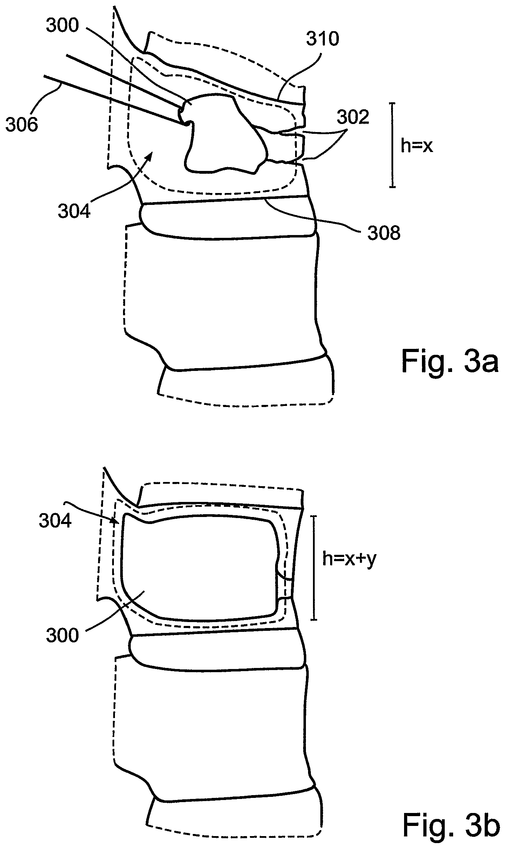

The expandable bone filler material may be injected (108) at a "low" pressure helping to avoid leakage of the bone filler material into undesired areas of the patient's body. The expandable quality of the bone filler material is optionally used to expand bone filler material into cracks, crevices and/or interstitial spaces at the treatment site, as shown in Figs. 3A-3B and 4A-4C, *in lieu* of high pressure (which is one technique used today for providing wide dispersion of bone cement in a space).

As described herein, high pressure injection carries with it the risk of leakage of the bone filler material into unintended, possibly unsafe, locations. Optionally the injection (108) pressure is low relative to the above-referenced conventional bone cement treatment procedures (e.g. vertebroplasty). Optionally, "low pressure is under 115 MPa (15 bar). Optionally, "low" pressure means 0.8-1.5 MPa (8-15 bar). Optionally, "low" pressure is under 1 MPa (10 bar). The "low" injection pressure is used without preparation of the treatment site prior to injection of the bone filler material, for example without using a kyphoplasty balloon, in a procedure which takes advantage of the expandable nature of the bone filler material, as described herein.

As described elsewhere herein, expansion of the bone filler material may be activated (110). Optionally, an attending medical professional activates (110) expansion of the bone filler material using a form of applied energy. In an embodiment of the invention, applied energy is at least one of the following: radiofrequency (RF), heat, light (coherent or broadband), including laser and IR, ultrasound, microwave, electrical and/or magnetic. In some embodiments of the invention, applied energy derives from the injection device (e.g. cannula or injection needle) and/or a tool 606 inserted with the injection device, as shown in Fig. 6B. Activation (110) is optionally halted upon the attainment of the desired expansion amount of the bone filler material. In some embodiments of the invention, the bone filler material is adapted such that the applied energy required to activate (110) its expansion is within a safe level. For example, using heat as applied energy doesn't burn the patient in order to activate (110) the bone filler material. Optionally, the application of applied energy is targeted such that the patient is not adversely effected by the energy, even if the energy is at a level which nominally would be dangerous to the patient.

In some embodiments of the invention, at least a portion of the applied energy is derived from the bone filler material itself, for example heat generated from the curing process. During the setting phase, the heat emitted from the exothermic curing process of the bone filler material may raise the filler material temperature to 70-140°C. Optionally, self-expansion occurs when the curing process of the acrylic (in embodiments where at least one base component is acrylic based) filler material reaches a minimal higher temperature, for example at the beginning of the setting phase. Optionally, the activation (110) temperature is higher than 37°C. Optionally, the activation (110) temperature is higher than 50°C. Optionally, the activation (110) temperature is higher than 70°C. Optionally, the activation (110) temperature is higher than 120°C. In an exemplary embodiment of the invention, the bone filler expansion process absorbs at least part of the heat emitted during the curing process so that the temperature surrounding the curing bone filler material remains relatively low. Optionally, the temperature surrounding the curing bone filler material is not substantially higher than 37°C.

In some embodiments of the invention, applied energy is derived from the body heat of the patient.

In some embodiments of the invention, at least a portion of the bone filler material contains air pockets and/or bubbles, that expand when heated, thus promoting expansion of at least a portion of the overall bone filler material mass. Applied energy is optionally used to activate (110) expansion of the air pockets and/or bubble.

Once injection (108) and/or optional activation (110) are performed, some or all injection devices and/or other instruments used for performing the procedure within the patient are withdrawn (112) from the patient, in an embodiment of the invention.

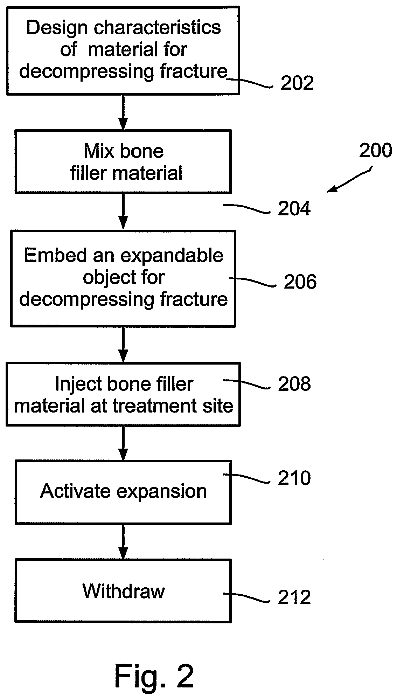

Fig. 2 shows a flowchart 200 depicting a method for treating a compression fracture. Optionally, the compression fracture is a vertebral compression fracture such as shown in Figs. 3A and 3B. Some or all of the actions of the method shown and described with respect to Fig. 1 are employed in a method for treating a compression fracture. For example, some or all of designing (202) the filler material, mixing (204) the material, embedding (206) an expandable object objects in the material, injecting (208) that material into a treatment site, activating (210) expansion of at least a portion of the bone filler material and/or withdrawing (212) treatment devices are optionally performed as described elsewhere herein.

In a method for treating a compression fracture, some actions are optionally tailored to address compression fracture issues. For example, designing (202) is used to define a bone filler material which will exhibit characteristics which will restore the compression fracture at least partially. An object or objects may be embedded (206) in the bone filler material which, upon expansion, will restore at least partially the compression fracture to a pre-fracture and/or healthy condition. Injection (208) is optionally performed to deposit the bone filler material in the most advantageous location or locations for restoring the compressed fracture.

Expandable bone filler material 300 can be used to treat a vertebral compression fracture 302, shown in Fig. 3A, by injecting the material with an injection device 306 into the interior 304 of the vertebra and expanding the material 300 to decompress the compression 302 (where the compressed vertebra has a height = x). The expansion of the bone filler material 300 in the vertebra interior 304 may restore the bone to at least a portion of its former, healthy height, shown in Fig. 3B (where height = x + y, y being some distance larger than 0). Optionally, the vertebra is restored to its full height prior to the compression fracture. The bone filler material may be injected (208) specifically towards the lower and/or upper vertebral end plate(s) 308, 310 respectively, in order to improve height restoration. The expandable bone filler material may be used to provide stability to an implant for treating the compression fracture located within the interior 304 of the vertebra by expanding around the implant and into the space between the implant and the bone. The bone filler material may be designed (202) to expand to decompress the vertebra until a predefined resistance is exerted on the bone filler material, at which point it expands and/or flows laterally (i.e. not in a direction which causes height enhancement of the vertebra).

The phrase "consisting essentially of means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

The word "optionally" is used herein to mean "is provided in some embodiments and not provided in other embodiments". Any particular embodiment of the invention may include a plurality of "optional" features unless such features conflict.

## Claims

1. A bone filler material for treating a compressed vertebra, comprising a mixture of:
acrylic base components, and
an additive which can provide an expansion force to at least a portion of the mixture, so as to expand the bone filler material to cause an increase in the height of a bone structure of the compressed vertebra surrounding the treatment site, in which the additive is air or discharges a gas to promote expansion of the bone filler material
in which the bone filler material goes through a polymerization process after mixing starts, so the mixed material becomes more viscous over time until it sets to full hardness.

2. The bone filler material according to claim 1, in which expansion of the additive increases a volume of the bone filler material by at least 5%.

3. The bone filler material according to claim 1 or 2, in which at least a portion of the additive contributes to expansion as a result of having energy applied to the mixture, preferably as at least one of heat, radio frequency, light, ultrasound, microwave, electrical or magnetic.

4. The bone filler material according to claim 1, in which the bone filler material is suitable for receiving the air from an injection device during injection into the treatment site.

5. The bone filler material according to claim 1, in which the air is provided as air pockets which are encased in a degradable material suitable for breaking down and allowing the air to be released to cause the bone filler material to expand.

6. The bone filler material according to claim 5, in which the degradable material comprises a polymer material with a T_{g} of less than 40°C suitable for changing into a liquid and releasing air upon heating.

7. The bone filler material according to claim 1, in which the additive is a biodegradable material which releases CO₂.

8. The bone filler material according to any of claims 1-7, in which expansion of the additive is suitable for increasing a volume of the bone filler material by at least 100%.

9. The bone filler material according to claim 1, in which the additive occupies between 5% and 45% of a total volume of the mixture.

10. The bone filler material according to claim 1, in which the additive occupies more than 45% of a total volume of the mixture.

11. The bone filler material according to claim 1, in which expansion of the additive increases a volume of the bone filler material by at least 100%.

12. The bone filler material according to claim 5, in which the degradable material comprises polyglycolic acid (PGA).

13. The bone filler material according to any of claims 1 to 12, wherein the acrylic base components comprise a polymethylmethacrylate powder and a liquid methylmethacrylate monomer.

## Patentansprüche

1. Knochenfüllmaterial zum Behandeln eines komprimierten Wirbels, umfassend eine Mischung aus:
Acrylbasiskomponenten und
ein Additiv, das eine Expansionskraft für mindestens einen Teil der Mischung bereitstellen kann, um das Knochenfüllmaterial auszudehnen, um eine Zunahme der Höhe einer Knochenstruktur des komprimierten Wirbels zu bewirken, welche die Behandlungsstelle umgibt, wobei das Additiv Luft ist oder ein Gas ausstößt, um die Ausdehnung des Knochenfüllmaterials zu fördern,
wobei das Knochenfüllermaterial nach dem Beginn des Mischens einen Polymerisationsprozess durchläuft, so dass das gemischte Material mit der Zeit viskoser wird, bis es die volle Härte erreicht hat.

2. Knochenfüllmaterial nach Anspruch 1, wobei die Ausdehnung des Additivs ein Volumen des Knochenfüllmaterials um mindestens 5% erhöht.

3. Knochenfüllmaterial nach Anspruch 1 oder 2, wobei zumindest ein Teil des Additivs zur Ausdehnung infolge der auf die Mischung aufgebrachten Energie beiträgt, vorzugsweise als zumindest eine von Wärme, Funkfrequenz, Licht, Ultraschall, Mikrowelle, elektrisch oder magnetisch.

4. Knochenfüllmaterial nach Anspruch 1, wobei das Knochenfüllmaterial geeignet ist zum Aufnehmen von Luft von einer Injektionsvorrichtung während der Injektion in die Behandlungsstelle.

5. Knochenfüllmaterial nach Anspruch 1, wobei die Luft als Lufttaschen bereitgestellt wird, die von einem abbaubaren Material ummantelt sind, das geeignet ist, sich zu zersetzen und der Luft erlaubt, freigesetzt zu werden, um das Knochenfüllmaterial zu veranlassen, sich auszudehnen.

6. Knochenfüllmaterial nach Anspruch 5, wobei das abbaubare Material ein Polymermaterial mit einer T_{g} von weniger als 40°C umfasst, das geeignet ist, sich in eine Flüssigkeit zu ändern und Luft beim Erwärmen freizusetzen.

7. Knochenfüllmaterial nach Anspruch 1, wobei das Additiv ein biologisch abbaubares Material ist, das CO₂ freisetzt.

8. Knochenfüllmaterial nach einem der Ansprüche 1-7, wobei die Ausdehnung des Additivs geeignet ist zum Erhöhen eines Volumens des Knochenfüllmaterials um mindestens 100 %.

9. Knochenfüllmaterial nach Anspruch 1, wobei das Additiv zwischen 5 % und 45 % eines Gesamtvolumens der Mischung einnimmt.

10. Knochenfüllmaterial nach Anspruch 1, wobei das Additiv mehr als 45 % eines Gesamtvolumens der Mischung einnimmt.

11. Knochenfüllmaterial nach Anspruch 1, wobei die Ausdehnung des Additivs ein Volumen des Knochenfüllmaterials um mindestens 100 % erhöht.

12. Knochenfüllmaterial nach Anspruch 5, wobei das abbaubare Material Polyglykoliksäure (PGA) umfasst.

13. Knochenfüllmaterial nach einem der Ansprüche 1 bis 12, wobei die Acrylbasiskomponenten ein Polymethylmethacrylat-Pulver und ein flüssiges Methylmethacrylat-Monomer umfassen.

## Revendications

1. Matériau de remplissage osseux pour traiter une vertèbre compressée, comprenant un mélange de :
composants de base acrylique, et
un additif qui peut fournir une force d'expansion à au moins une partie du mélange, de manière à étendre le matériau de remplissage osseux afin d'entraîner une augmentation de la hauteur d'une structure osseuse de la vertèbre compressée entourant le site de traitement, dans lequel l'additif est de l'air ou rejette un gaz pour favoriser l'expansion du matériau de remplissage osseux
dans lequel le matériau de remplissage osseux passe par un processus de polymérisation après le démarrage du mélange, de sorte que le matériau mélangé devient plus visqueux avec le temps jusqu'à ce qu'il prenne prise à dureté complète.

2. Matériau de remplissage osseux selon la revendication 1, dans lequel l'expansion de l'additif augmente un volume du matériau de remplissage osseux d'au moins 5 %.

3. Matériau de remplissage osseux selon la revendication 1 ou 2, dans lequel au moins une partie de l'additif contribue à l'expansion à la suite de l'application d'énergie au mélange, de préférence telle qu'au moins une parmi l'énergie thermique, à radiofréquence, lumineuse, ultrasonore, à micro-ondes, électrique ou magnétique.

4. Matériau de remplissage osseux selon la revendication 1, dans lequel le matériau de remplissage osseux est approprié pour recevoir l'air provenant d'un dispositif d'injection durant l'injection dans le site de traitement.

5. Matériau de remplissage osseux selon la revendication 1, dans lequel l'air est fourni sous forme de poches d'air qui sont enfermées dans un matériau dégradable approprié pour se rompre et permettre à l'air d'être libéré pour entraîner l'expansion du matériau de remplissage osseux.

6. Matériau de remplissage osseux selon la revendication 5, dans lequel le matériau dégradable comprend un matériau polymère ayant une T_{g} de moins de 40 °C approprié pour se changer en un liquide et libérer de l'air lors du chauffage.

7. Matériau de remplissage osseux selon la revendication 1, dans lequel l'additif est un matériau biodégradable qui libère du CO₂.

8. Matériau de remplissage osseux selon l'une quelconque des revendications 1-7, dans lequel l'expansion de l'additif est appropriée pour augmenter un volume du matériau de remplissage osseux d'au moins 100 %.

9. Matériau de remplissage osseux selon la revendication 1, dans lequel l'additif occupe entre 5 % et 45 % d'un volume total du mélange.

10. Matériau de remplissage osseux selon la revendication 1, dans lequel l'additif occupe plus de 45 % d'un volume total du mélange.

11. Matériau de remplissage osseux selon la revendication 1, dans lequel l'expansion de l'additif augmente un volume du matériau de remplissage osseux d'au moins 100 %.

12. Matériau de remplissage osseux selon la revendication 5, dans lequel le matériau dégradable comprend de l'acide polyglycolique (PGA).

13. Matériau de remplissage osseux selon l'une quelconque des revendications 1 à 12, dans lequel les composants de base acrylique comprennent une poudre de polyméthylméthacrylate et un monomère de méthylméthacrylate liquide.
